# EUROPEAN PATENT SPECIFICATION

(11) **EP 3 777 786 B1**
(45) Date of publication and mention of the grant of the patent: **07.06.2023**
(21) Application number: 20191129.4
(22) Date of filing: 14.08.2020
(51) Int. Cl.: A61F 5/56

(54) **NASAL BREATHING TRAINING TAPE AND METHOD**
NASALES ATMUNGSTRAININGSBAND UND VERFAHREN
BANDE D'ENTRAÎNEMENT ET PROCÉDÉ DE RESPIRATION NASALE

(30) Priority: 16.08.2019 US 201916542363
(43) Date of publication of application: 17.02.2021
(73) Proprietor: McKeown, Patrick, Moycullen H91 H4C1 Galway (IE)
(72) Inventor: McKeown, Patrick, Moycullen H91 H4C1 Galway (IE)
(74) Representative: Butler, Kathryn Louise

(56) References cited:
- WO-A1-99/61089
- WO-A1-2017/218051
- US-A1- 2016 338 870

## Description

### FIELD OF THE INVENTION

The present invention generally relates to training equipment, and, more particularly, to a training equipment that imparts training to children, teens and adults to breathe from the nose and not from the mouth, during sleep time or wakefulness time.

### BACKGROUND OF THE INVENTION

The inventor, Patrick McKeown has written seven books on breathing re-education for children, teenagers and adults. The foundation of breathing re-education is to stop the habit of breathing through the mouth during wakefulness and sleep and change to nasal breathing only. Human beings, particularly children and teenagers, have a common tendency to breathe from their mouth rather than their nose, during wakefulness while performing various day to day activities, such as playing, sitting, watching television or during sleep. Up to 50% of studied children persistently breathe through an open mouth. ( Felcar, Josiane Marques, Izabele Rafael Bueno, Ana Carolina Silva Massan, Roberta Pereira Torezan, and Jefferson Rosa Cardoso. "Prevalence of mouth breathing in children from an elementary school." Ciencia & saude coletiva 15, no. 2 (2010): 427 .) Breathing through the mouth is not natural and can cause various problems to both children and adults. For example, mouth breathing can contribute to crooked teeth, facial deformities, drooling, or poor growth of children. In adults, chronic mouth breathing can cause bad breath, gum disease and can worsen symptoms of other illnesses including asthma and sleep apnea. ( McKeown P, The Oxygen Advantage. Little Brown Press. September 2015 )

Mouth breathing during sleep is significantly associated with worse oxygen desaturation and an increased degree of upper airway collapse in adults. ( Yen-Bin Hsu, Association Between Breathing Route, Oxygen Desaturation, and Upper Airway Morphology. DOI: 10.1002/lary.28774)

Accordingly, there exists a need to impart training to children, teenagers or adults to breathe from the nose and not from the mouth, during sleep time and wakefulness.

### SUMMARY OF THE INVENTION

In view of the foregoing disadvantages inherent in the prior art, the general purpose of the present invention is to provide a nasal breathing training tape and method to overcome the drawbacks inherent in the prior art.

An object of the present disclosure is to impart training skills to children, teenagers or adults to breathe from the nose and not from the mouth, during sleep time or wakefulness.

With that regard, in one aspect of the present invention, a nasal breathing training tape is provided as defined in claim 1. The stretchable strip has sufficient stretch to create tension therein. The stretchable strip is stretched to a size to be accommodated around, and to stick around, upper and lower lips of the user, surrounding the mouth but not touching the upper and lower lips of the user, such that the upper and lower lips are brought together by the tension created in the stretchable strip. Further, to obtain habitual mouth closure and nasal breathing, for many children and adults it is necessary to strengthen the orbicularis oris muscle. While wearing the nasal breathing training tape, the user feels the orbicular muscle oris contract, causing the lips to close. The tension created by the nasal breathing training tape, an overall neuro-sensory-motor system of the user is stimulated, so that the brain of the user learns to 'switch on' the nasal breathing, when the upper and lower lips are gently held together by the stretchable strip. In addition, the tape can be worn during the day as the user can speak and open their mouth while wearing the tape.

In another aspect of the present invention, a method for imparting training to a user to breathe from the nose and not from the mouth, during sleep time or wakefulness is provided as defined in claim 8. Due to the tension created by the nasal breathing training tape, an overall neuro-sensory-motor system of the user is stimulated, so that the brain of the user learns to 'switch on' the nasal breathing, when the upper and lower lips are gently held together.

This together with the other aspects of the present invention, along with the various features of novelty that characterize the present invention, is pointed out with particularity in the claims annexed hereto and forms a part of the present invention. For a better understanding of the present invention, its operating advantages, and the specified object attained by its uses, reference should be made to the accompanying drawings and descriptive matter in which there are illustrated exemplary embodiments of the present invention.

### BRIEF DESCRIPTION OF THE DRAWINGS

The advantages and features of the present invention will become better understood with reference to the following detailed description and claims taken in conjunction with the accompanying drawings, wherein like elements are identified with like symbols, and in which:
- FIG. 1 illustrates an environment wherein a nasal breathing training tape is shown to be incorporated surrounding the users' mouth to impart training to a user to breathe from the nose and not from the mouth, during sleep time or wakefulness time, in accordance with an exemplary embodiment of the present disclosure;
- FIGS. 2A to 2C illustrate various views of a nasal breathing training tape, i.e. FIG. 2A illustrates top view of the nasal breathing training tape, FIG. 2B illustrates back view of the nasal breathing training tape with an adhesive layer, and FIG. 2C illustrates back view of the nasal breathing training tape with a layer covering the adhesive layer which is peeled off to allow the nasal breathing strip to be affixed by way of the adhesive, in accordance with an exemplary embodiment of the present disclosure;
- FIG. 3 illustrates an example of dimensional measurement of a nasal breathing training tape, in accordance with an exemplary embodiment of the present disclosure;
- FIG. 4 illustrates an example of shape and dimensional measurement of a nasal breathing training tape, in accordance with another exemplary embodiment of the present disclosure;
- FIG. 5 illustrates a nasal breathing training tape having patterns, in accordance with another exemplary embodiment of the present disclosure; and
- FIG. 6 illustrates a flow chart of a method of using a nasal breathing training tape, in accordance with another exemplary embodiment of the present disclosure.

Like reference numerals refer to like parts throughout the description of several views of the drawings.

### DETAILED DESCRIPTION OF THE INVENTION

For a thorough understanding of the present invention, reference is to be made to the following detailed description, including the appended claims, in connection with the above-described drawings. Although the present invention is described in connection with exemplary embodiments, the present invention is not intended to be limited to the specific forms set forth herein. It is understood that various omissions and substitutions of equivalents are contemplated as circumstances may suggest or render expedient, but these are intended to cover the application or implementation without departing from the scope of the claims of the present invention. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

The terms, "a" and "an" herein do not denote a limitation of quantity, but rather denote the presence of at least one of the referenced items.

The present invention provides a nasal breathing training tape as defined in claim 1. The nasal breathing training tape is adapted to impart training to a user to breathe from the nose, and not from the mouth, during sleep time or wakefulness time. The nasal breathing training tape includes a stretchable strip, an adhesive layer, and a cutout portion. The stretchable strip includes a first surface, and a second surface placed on top of the first surface. The stretchable strip is stretchable to create tension therein. Further, the adhesive layer may be applied to the second surface of the stretchable strip. Furthermore, the cutout portion is configured on the stretchable strip at a substantially central location of the stretchable strip. The stretchable strip is stretched to a size to be accommodated and stuck around a upper and lower lips, surrounding the mouth but not touching the upper and lower lips, of the user, such that the upper and lower lips are held together by the tension created in the stretchable strip. Further, due to the tension created by the nasal breathing training tape, an overall neuro-sensory-motor system of the user is stimulated, so that the brain of the user learns to 'switch on' the nasal breathing, when the upper and lower lips are gently held together by the stretchable strip.

The present invention also provides a method as defined in claim 8 for imparting training to a user to breathe from the nose, and not from the mouth, during sleep time or wakefulness time. The method includes stretching a stretchable strip of a nasal breathing training tape up to a size to be accommodated around upper and lower lips of the user, and to create tension therein. The stretchable strip includes a cutout portion configured on the stretchable strip along a substantially central location of the stretchable strip. Further, the method includes sticking the stretched stretchable strip, via an adhesive layer applied to a second surface and placed on top of a first surface of the stretchable strip, around the upper and lower lips, surrounding the mouth but not touching the upper and lower lips of the user, such that the upper and lower lips are held together by the tension created in the stretchable strip. Due to the tension created by the nasal breathing training tape, an overall neuro-sensory-motor system of the user is stimulated, so that the brain of the user learns to 'switch on' the nasal breathing, when the upper and lower lips are gently held together.

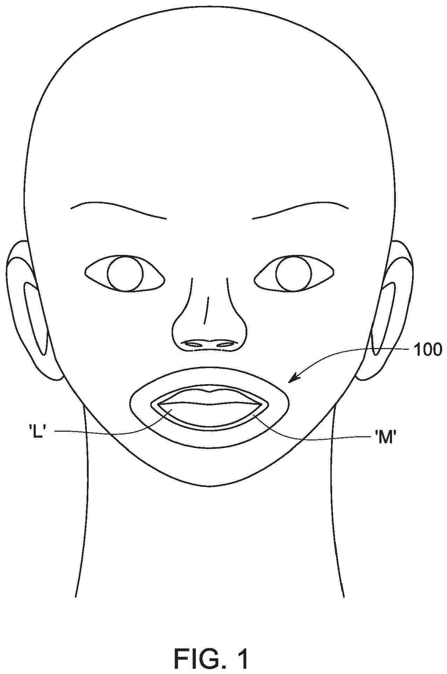

Referring now to FIG. 1 , a nasal breathing training tape 100 is shown to be incorporated around a user's mouth 'M' to impart training to the user to breathe from the nose, and not from the mouth, during sleep time or wakefulness time, in accordance with an exemplary embodiment of the present disclosure. Specifically, nasal breathing training tape 100 may be stretched to a size to be accommodated and to stick around upper and lower lips 'L', surrounding the mouth 'M' but not touching the upper and lower lips 'L', of the user, such that the upper and lower lips 'L' are held together.

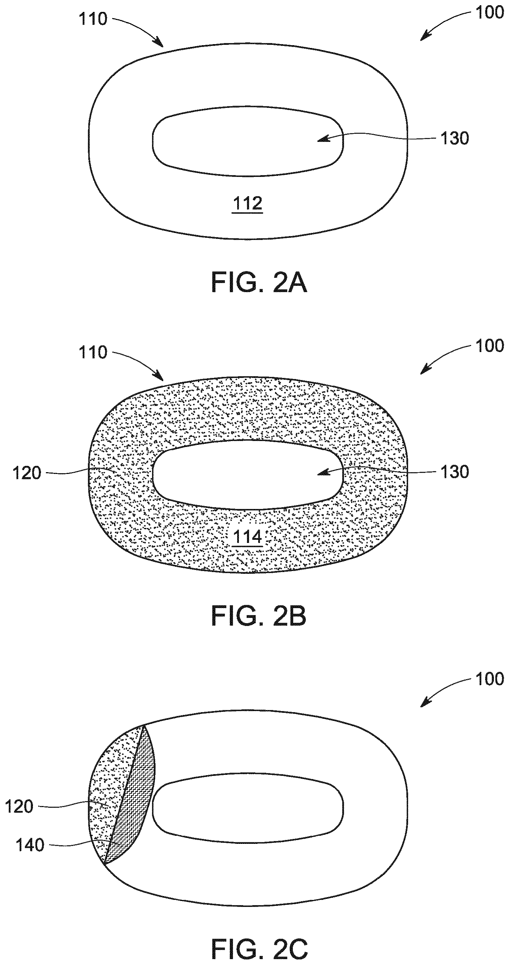

Referring now to FIGS. 2A to 2C , various views of the nasal breathing training tape 100, in accordance with an exemplary embodiment of the present disclosure are shown and now will be explained in conjunction with FIG. 1. In one example embodiment, as shown in FIGS. 2A to 2C, the nasal breathing training tape 100 may include a stretchable strip 110, an adhesive layer 120, and a cutout portion 130. The stretchable strip 110 may include a first surface 112, and a second surface 114 opposite to the first surface 112. The stretchable strip 110 may be stretchable to create tension therein. Further, the adhesive layer 120 may be applied to the second surface 114 of the stretchable strip 100. Furthermore, the cutout portion 130 may be disposed along a substantially central location of the stretchable strip 110. In one embodiment, the cutout portion 130 generally corresponds to the upper and lower lips 'L', such that the upper and lower lips 'L' protrudes from the cutout portion 130, as shown in FIG. 1 .

The stretchable strip 110 may be stretched to a size to be accommodated and to be stuck around the upper and lower lips 'L', surrounding the mouth 'M' but not touching the upper and lower lips 'L', of the user, such that the upper and lower lips 'L' are held together by the tension created in the stretchable strip 110. Further, due to the tension created by the nasal breathing training tape 100, an overall neuro-sensory-motor system of the user is stimulated, so that the brain of the user learns to 'switch on' the nasal breathing, when the upper and lower lips 'L' are gently held together by the stretchable strip 110.

The stretchable strip 110 is made of a cotton material and a synthetic material that has a stretchable and elastic property. In one preferred embodiment of the present disclosure, the stretchable strip 110 may include a property of stretchability of about 20% to about 30%. For example, the stretchable strip 110 may be stretched to about 20% to about 30% of its actual size to create tension in the stretchable strip 110. Stretchability may be determined as described below, based on a change in dimension when a force of 300 grams is applied from an original rest condition of the stretchable strip 110 until resistance is encountered to substantially stop the stretching. The adhesive layer 120 also retains its adhesiveness when the stretchable strip 110 is stretched to about 20% to 30%. When released, the stretchable strip 110 will recover most of its original dimensions under elastic property of the strip 110. In one example, a stretchable strip 110 of about 54 mm (in a direction from a left side to a right side of the lips, perpendicular to a mouth opening direction) may stretch to about 65 mm, at which the adhesiveness of the adhesive layer 120 is also effectively retained.

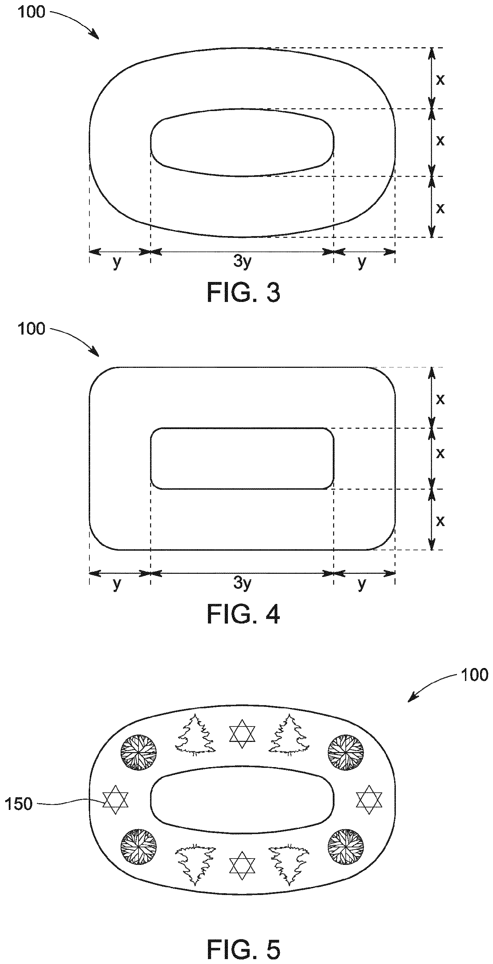

The stretchable strip 110 may be one of a rectangular shape or of an elliptical shape (with respect to an outer perimeter). One example depiction of the elliptical shape of the stretchable strip is shown in FIGS. 2A to 2C . However, the stretchable strip 110 may also be of the rectangular shape (with curved corners and straight sides), such as shown in FIG. 4 . Where the stretchable strip 110 is rectangular, the corners thereof may be rounded, as shown in FIG. 4 . Further, the cutout portion 130 may also correspond to the shape of the stretchable strip 110, and may be one of a rectangular shape, as shown in FIG. 4 , and may be of an elliptical shape, as shown in FIGS. 2A-2C . Where the shape of the cutout portion 130 is rectangular, the corners thereof may also be rounded, as shown in FIG. 4 . As shown in FIGS. 2A-2C or FIG. 4 , the shape of the stretchable strip 110 and the cutouts 130 are elliptical or rectangular in these examples. However, the shape of the stretchable strip 110 and the cutouts 130 are not considered to be limited only to elliptical or rectangular. The disclosure, without departing from the scope of the present disclosure, intends to cover various other shapes, such as circular or square and so forth. However, the outer perimeter of the stretchable strip 110 and the shape of the cutout portion 130 will generally follow the shape of the upper and lower lips of the user.

The stretchable strip 110 comprises a predetermined shape having a height (in a direction of opening and closing of the mouth) and a width (in a direction from left to right sides of the mouth of the user), wherein a ratio of height to width is about 3:5. For example, as shown in FIG. 3 , the stretchable strip 110 has a predetermined shape of an ellipse having a height of 3x and a width of 5y, wherein x=y=1. Another example is shown in FIG. 4, where the stretchable strip 110 has a predetermined shape of a rectangle having a height of 3x and a width of 5y, wherein x=y=1. In one example, the stretchable strip 110 may have a width of about 54 mm and a height of about 33 mm. In one another example, the stretchable strip 110 may have a height of about 30 mm and the width of about 50mm.

The area ratio of the cutout portion 130 to the stretchable strip 110 is 1:3. For example, if the stretchable strip 110 has a width of 5y and height of 3x; then the cutout portion 130 may be of 1x in height and 3y in width, wherein x=y=1. In a further example, if the stretchable strip 110 has a width of about 54 mm and a height of about 33 mm, then the cutout portion 130 may have a width of about 32 mm and a height of about 11 mm. In a further example, if the stretchable strip 110 has a height of about 30 mm and a width of about 50mm, then the cutout portion 130 may have a width of about 30 mm and a height of about 10 mm.

The cutout portion 130 has a width of between 30mm and 45 mm and a height of between 10 mm and 20 mm. Different size nasal breathing training tapes 100 may be provided depending on the size of the mouth, whether the wearer is adult or child, etc. The border of material around the cutout portion 130 should be about 20 mm to 35 mm when measuring from the cutout portion 130 to the outside of the stretchable strip 110 along a shortest perpendicular line (along linear portions) or radial line (at corner portions).

In embodiments, the stretchability described herein is determined by gently pulling the nasal breathing training tape 100 until there is a clear resistance to further stretching and measuring the before and after dimensions. A more precise testing method is not required as precise stretchabilities are not being recited. The nasal breathing training tape 100 may be stretch between 110% and 140% at least in the width direction (in the direction of the left and right sides of the mouth in use) and optionally also in the height direction (the mouth opening and closing direction) when gently pulled by an adult user until resistance. The force applied may be 300 gram force.

The stretchable strip 110 may be stretchable by the user in at least the horizontal direction and/or the vertical direction. The stretchable strip should additionally be elastic to apply a force/tension to the lips when used. In embodiments, the stretchable strip 110 is a fibrous material. The stretchable strip 110 is made of a blend of cotton and synthetic fiber. In one embodiment, cotton and spandex fibers are blended. Other blends include Cotton, Rayon and/or Nylon. The fibers may be woven. The stretchability and elasticity are significantly greater in the horizontal direction than the vertical direction. This is achieved by horizontally orienting a majority of the fibers. The cotton and synthetic threads are included into a weft yarn to provide a uni-directional elasticity and stretch in a horizontal direction along the width of the mouth.

In one further embodiment, the stretchable strip 110 may include one or more patterns of cartoon characters, or, one or more universal characters including cars, stars, moon, sun, flowers, or any other graphical elements, and so forth, as shown in FIG. 5 . Particularly, the first surface 112 of the stretchable strip 110 may include such cartoon characters and/or universal characters. Such cartoon characters and/or universal characters may make the nasal breathing training tape 100 attractive to children and children may get encouraged to stick the nasal breathing training tape for training purposes.

In one embodiment, as mentioned above, the adhesive layer 120 may be applied to the second surface 114 of the stretchable strip 110. The adhesive in the adhesive layer 120 is such that it is adaptable to human skin. In one embodiment, as shown in FIG. 2B , the nasal breathing training tape 100 may be directly stretched and stuck to surround the mouth 'M' but not touching the upper and lower lips 'L', with the help of an exposed adhesive layer 120. Such adhesive may retain its adhesiveness upon applying a liquid to the adhesive layer 120. However, in another embodiment, as shown in FIG. 2C , the adhesive layer 120 may be covered by another layer, such as layer 140, made of paper or plastic. In this embodiment, the user may remove the layer 140 to expose the adhesive layer 120. Upon exposure of the adhesive layer 120, the nasal breathing training tape 100 may be directly stretched and stuck to surround the mouth 'M' but not touching the upper and lower lips 'L'.

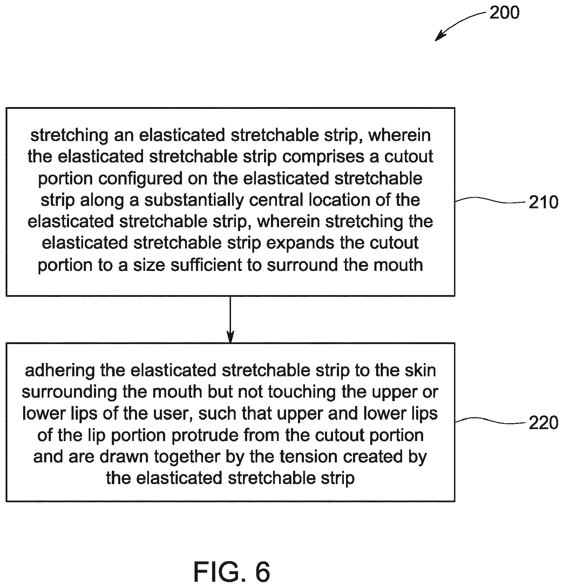

Referring now to FIG. 6 , a method 200 for imparting training to the user to breathe from the nose, and not from the mouth, during sleep time or wakefulness time is provided. At step 210, the stretchable strip 110 of the nasal breathing training tape 100 is stretched to a size to be accommodated around upper and lower lips of the user, and to create tension therein. The tension is created by elastic properties of the tape. The tension may be created in the up and down direction (the opening and closing direction of the mouth), the left and right direction (across the mouth) or both. In one embodiment, the stretchable strip may be stretched from about 20% to about 30% additional to the original length. Further, at step 220, the method 200 includes sticking the stretched stretchable strip, via the adhesive layer 120, so as to surround the mouth but not touching the upper and lower lips, of the user, such that the upper and lower lips are held together by the tension created in the stretchable strip 110. Due to the tension created by the nasal breathing training tape 100, an overall neuro-sensory-motor system of the user is stimulated, so that the brain of the user learns to 'switch on' the nasal breathing, when the upper and lower lips are gently held together. In one embodiment, the nasal breathing training tape 100 may be worn during the day for about 30 minutes to two hours, especially while a child (or adult) is distracted. The nasal breathing training tape 100 may be worn during sleep for a more extended period of time.

The present invention provides a nasal breathing training tape, such as the nasal breathing training tape 100 or 300, which offers the various advantages. Most importantly, the nasal breathing training tape 100, 300 imparts training skills to children, teen or adults to breathe from the nose, and not from the mouth, during sleep time or wakefulness time. The nasal breathing training tape is placed surrounding the lips, but not in direct contact with the lips. The nasal breathing training tape may be made from cotton or synthetic materials to stretch in two or four ways. Due to the tension created by the nasal breathing training tape, the overall neuro-sensory-motor system is stimulated, so that the brain learns to 'switch on' nasal breathing when the lips are gently held together. The nasal breathing training tape stimulates the skin, which is regulated by the trigeminal, which sandwiches the perioral muscles, including the orbicularis oris regulated by the facial muscles. The purpose of the nasal breathing training tape is to re-educate children and teenagers to nasal breathe. The nasal breathing training tape should be worn during the day for 30 minutes to two hours, especially while child is distracted. The tape may also be worn during sleep. The nasal breathing training tape described herein may additionally find application in sports to encourage wearers to breathe properly. Athletes can wear the tape while partaking in physical exercise. Since the tape surrounds the mouth, the athlete can drink from a bottle as well as communicate.

The foregoing descriptions of specific embodiments of the present invention have been presented for purposes of illustration and description. They are not intended to be exhaustive or to limit the present invention to the precise forms disclosed, and obviously many modifications and variations are possible in light of the above teaching. The embodiments were chosen and described in orderto best explain the principles of the present invention and its practical application, to thereby enable others skilled in the art to best utilize the present invention and various embodiments with various modifications as are suited to the particular use contemplated. It is understood that various omission and substitutions of equivalents are contemplated as circumstance may suggest or render expedient, but such are intended to cover the application or implementation without departing from the scope of the claims of the present invention.

## Claims

1. A nasal breathing training tape to impart training to a user to breathe from the nasal, and not from the mouth, during sleep time or wake-up time, the nasal breathing training tape comprising:
a stretchable strip comprising fibrous material comprising a blend of cotton with synthetic fibre selected from spandex, rayon and/or nylon, in which the cotton and synthetic fibre are included into a weft yarn to provide a uni-directional elasticity and stretch in a horizontal direction along the width of the mouth of the user, in which the stretchable strip has a first surface, and a second surface opposite to the first surface, wherein the stretchable strip is stretchable to create tension therein, wherein the stretchable strip has a height to width ratio of 3:5, in which the height of the stretchable strip is between 50 mm and 54 mm and the width of the stretchable strip is between 30 mm and 33 mm,
an adhesive layer applied to the second layer; and
a cutout portion disposed along a substantially central location of the stretchable strip,
in which the area ratio of the cutout portion to the stretchable strip is 1:3,
wherein the stretchable strip is stretched up to a size to be accommodated and stuck around upper and lower lips surrounding the mouth of a user but not in direct contact with the lips, wherein the cutout portion corresponds to the lip portion of a user such that in use the lip portion protrudes from the cutout portion to enable the user to speak and open the mouth during use,
and in which the training tape is positioned in use to be positioned adjacent to the orbicularis oris muscle, and in which the tension created in the training tape causes contraction of the orbicularis oris muscle causing the lips to close and be held together.

2. The nasal breathing training tape of claim 1, wherein the stretchable strip comprises one of a rectangular shape and an elliptical shape, wherein the stretchable strip with the rectangular shape having rounded corners.

3. The nasal breathing training tape of claim 1, wherein the stretchable strip comprises a stretchability of about 20% to about 30%.

4. The nasal breathing training tape of claim 1, wherein the first surface of the stretchable strip comprises one or more patterns of cartoon characters, or, one or more universal characters including cars, stars, moon, sun, flowers, and so forth.

5. The nasal breathing training tape of claim 1, wherein the cutout portion corresponds to the lip portion such that the lip portion protrudes from the cutout portion.

6. The nasal breathing training tape of claim 1, wherein the cutout portion comprises one of a rectangular shape and an elliptical shape, wherein the cutout portion with the rectangular shape comprises rounded corners.

7. The nasal breathing training tape of claim 1, wherein the adhesive is adaptable to human skin.

8. A method for imparting training to a user to breathe from the nasal, and not from the mouth, during sleep time or wake-up time using a nasal breathing training tape as claimed in any one of claims 1 to 7, the method comprising:
stretching a stretchable strip of the nasal breathing training tape up to a size to be accommodated around a lip portion of the user, and to create tension therein; and
sticking the stretched stretchable strip, via an adhesive layer, around the lip portion, surrounding the mouth such that the nasal breathing training tape is not touching the lip portion, of the user, such that the lip portion held together by the tension created in the stretchable strip.

9. The method of claim 8, wherein stretching the stretchable strip to about 20% to about 30%.

10. The method of claim 8, wherein sticking the stretched stretchable strip to about 30 minutes to 2 hours.

## Patentansprüche

1. Ein Nasenatmungs-Trainingsband, um einem Benutzer Training zum Atmen durch die Nase und nicht durch den Mund während des Schlafs und im Wachzustand zu vermitteln, wobei das Nasenatmungs-Trainingsband Folgendes beinhaltet:
einen dehnbaren Streifen, der ein faseriges Material beinhaltet, das eine Mischung aus Baumwolle mit synthetischen Fasern beinhaltet, ausgewählt aus Spandex, Rayon und/oder Nylon, wobei die Baumwolle und die synthetischen Fasern in einem Schussgarn eingeschlossen sind, um eine unidirektionale Elastizität und Dehnung in einer horizontalen Richtung entlang der Breite des Munds des Benutzers bereitzustellen, wobei der dehnbare Streifen eine erste Oberfläche und eine zweite, der ersten Oberfläche gegenüberliegende Oberfläche beinhaltet, wobei der dehnbare Streifen dehnbar ist, um Spannung darin zu erzeugen, wobei der dehnbare Streifen ein Verhältnis der Höhe zur Breite von 3:5 aufweist, wobei die Höhe des dehnbaren Streifens zwischen 50 mm und 54 mm und die Breite des dehnbaren Streifens zwischen 30 mm und 33 mm beträgt,
eine auf die zweite Oberfläche aufgetragene Klebeschicht; und
einen ausgeschnittenen Anteil, der entlang einer im Wesentlichen zentralen Stelle des dehnbaren Streifens angeordnet ist, wobei das Flächenverhältnis des ausgeschnittenen Anteils zu dem dehnbaren Streifen 1:3 beträgt,
wobei der dehnbare Streifen bis zu einer jeweils passenden Größe gedehnt wird und so um die Ober- und Unterlippen geklebt wird, dass er den Mund eines Benutzers umgibt, aber nicht in direktem Kontakt mit den Lippen ist, wobei der ausgeschnittene Anteil dem Lippenanteil eines Benutzers entspricht, sodass der Lippenanteil im Gebrauch aus dem ausgeschnittenen Anteil hervorragt, um es dem Benutzer zu ermöglichen, während der Benutzung zu sprechen und den Mund zu öffnen,
und wobei das Trainingsband im Gebrauch so positioniert wird, dass es neben dem Musculus orbicularis oris positioniert ist, und wobei die in dem Trainingsband erzeugte Spannung die Kontraktion des Musculus orbicularis oris auslöst, was dazu führt, dass die Lippen geschlossen werden und zusammengehalten werden.

2. Nasenatmungs-Trainingsband nach Anspruch 1, wobei der dehnbare Streifen einen mit rechteckiger Gestalt und einen mit elliptischer Gestalt beinhaltet, wobei der dehnbare Streifen mit der rechteckigen Gestalt gerundete Ecken aufweist.

3. Nasenatmungs-Trainingsband nach Anspruch 1, wobei der dehnbare Streifen eine Dehnbarkeit von etwa 20% bis etwa 30% beinhaltet.

4. Nasenatmungs-Trainingsband nach Anspruch 1, wobei die erste Oberfläche des dehnbaren Streifens ein oder mehrere Muster von Zeichentrickfiguren oder eine oder mehrere universelle Figuren beinhaltet, einschließlich Autos, Sterne, Mond, Sonne, Blumen und so weiter.

5. Nasenatmungs-Trainingsband nach Anspruch 1, wobei der ausgeschnittene Anteil dem Lippenanteil entspricht, sodass der Lippenanteil aus dem ausgeschnittenen Anteil hervorragt.

6. Nasenatmungs-Trainingsband nach Anspruch 1, wobei der ausgeschnittene Anteil einen mit einer rechteckigen Gestalt und einer elliptischen Gestalt beinhaltet, wobei der ausgeschnittene Anteil mit der rechteckigen Gestalt gerundete Ecken beinhaltet.

7. Nasenatmungs-Trainingsband nach Anspruch 1, wobei der Kleber an menschliche Haut adaptierbar ist.

8. Verfahren zum Vermitteln von Training an einen Benutzer zum Atmen durch die Nase und nicht durch den Mund während des Schlafs oder im Wachzustand unter Verwendung eines Nasenatmungs-Trainingsbands nach einem der Patentansprüche 1 bis 7, wobei das Verfahren Folgendes beinhaltet:
Dehnen eines dehnbaren Streifens des Nasenatmungs-Trainingsband bis zu einer jeweils passenden Größe um einen Lippenanteil des Benutzers und Erzeugen von Spannung darin; und
Kleben des gedehnten dehnbaren Streifens anhand einer Klebeschicht um den Lippenanteil herum, wobei der Mund so umgeben wird, dass das Nasenatmungs-Trainingsband den Lippenanteil des Benutzers nicht berührt, sodass der Lippenanteil durch die in dem dehnbaren Steifen erzeugte Spannung zusammengehalten wird.

9. Verfahren nach Anspruch 8, wobei das Dehnen des dehnbaren Streifens zu etwa 20% bis etwa 30% erfolgt.

10. Verfahren nach Anspruch 8, wobei das Kleben des gedehnten dehnbarem Streifens etwa 30 Minuten bis 2 Stunden lang erfolgt.

## Revendications

1. Bande d'entraînement à la respiration nasale, destinée à donner à un utilisateur un entraînement à respirer par voie nasale, et non par la bouche, pendant le sommeil ou le réveil, la bande d'entraînement à la respiration nasale comprenant :
une bande extensible comprenant un matériau fibreux comprenant un mélange de coton et de fibres synthétiques sélectionnées parmi l'élasthanne, la rayonne et/ou le nylon, dans laquelle le coton et les fibres synthétiques sont incorporés dans un fil de trame pour apporter une élasticité unidirectionnelle et s'étirent dans une direction horizontale le long de la largeur de la bouche de l'utilisateur, la bande extensible ayant une première surface, et une deuxième surface opposée à la première surface, la bande extensible étant extensible pour y créer une tension, la bande extensible ayant un rapport de la hauteur à la largeur de 3:5, la hauteur de la bande extensible étant comprise entre 50 mm et 54 mm et la largeur de la bande extensible étant comprise entre 30 mm et 33 mm,
une couche adhésive appliquée sur la deuxième surface ; et
une partie découpée disposée le long d'un emplacement sensiblement central de la bande extensible,
dans laquelle le rapport surfacique de la partie découpée à la bande extensible est de 1:3,
dans laquelle la bande extensible est étirée jusqu'à une taille lui permettant d'être reçue et collée autour des lèvres supérieure et inférieure entourant la bouche d'un utilisateur mais pas en contact direct avec les lèvres, la partie découpée correspondant à la région des lèvres d'un utilisateur de telle sorte que, en cours d'utilisation, la région des lèvres dépasse de la partie découpée pour permettre à l'utilisateur de parler et d'ouvrir la bouche pendant l'utilisation,
et dans laquelle la bande d'entraînement est placée en cours d'utilisation pour être positionnée de façon adjacente au muscle orbiculaire de la bouche, et la tension créée dans la bande d'entraînement provoquant une contraction du muscle orbiculaire de la bouche entraînant la fermeture des lèvres et leur maintien l'une contre l'autre.

2. Bande d'entraînement à la respiration nasale selon la revendication 1, dans laquelle la bande extensible comporte l'une d'une forme rectangulaire et d'une forme elliptique, la bande extensible de forme rectangulaire comportant des coins arrondis.

3. Bande d'entraînement à la respiration nasale selon la revendication 1, dans laquelle la bande extensible a une extensibilité d'environ 20 % à environ 30 %.

4. Bande d'entraînement à la respiration nasale selon la revendication 1, dans laquelle la première surface de la bande extensible comprend un ou plusieurs motifs constitués de personnages de dessins animés, ou un ou plusieurs éléments universels comprenant des voitures, des étoiles, la lune, le soleil, des fleurs, etc.

5. Bande d'entraînement à la respiration nasale selon la revendication 1, dans laquelle la partie découpée correspond à la région des lèvres de telle sorte que la région des lèvres dépasse de la partie découpée.

6. Bande d'entraînement à la respiration nasale selon la revendication 1, dans laquelle la partie découpée comporte l'une d'une forme rectangulaire et d'une forme elliptique, la partie découpée de forme rectangulaire comportant des coins arrondis.

7. Bande d'entraînement à la respiration nasale selon la revendication 1, dans laquelle l'adhésif est adaptable à la peau humaine.

8. Procédé permettant de donner à un utilisateur un entraînement à respirer par voie nasale, et non par la bouche, pendant le sommeil ou le réveil, à l'aide d'une bande d'entraînement à la respiration nasale selon l'une quelconque des revendications 1 à 7, le procédé comprenant :
l'étirement d'une bande extensible de la bande d'entraînement à la respiration nasale jusqu'à une taille lui permettant d'être reçue autour d'une région des lèvres de l'utilisateur, et pour y créer une tension ; et
le collage de la bande extensible étirée, par l'intermédiaire d'une couche adhésive, autour de la région des lèvres, en entourant la bouche de telle sorte que la bande d'entraînement à la respiration nasale ne touche pas la région des lèvres de l'utilisateur, de telle sorte que la région des lèvres soit maintenue fermée par la tension créée dans la bande extensible.

9. Procédé selon la revendication 8, dans lequel la bande extensible est étirée jusqu'à environ 20 % à environ 30 %.

10. Procédé selon la revendication 8, dans lequel la bande extensible étirée est collée pendant un maximum d'environ 30 minutes à 2 heures.
